# EUROPEAN PATENT SPECIFICATION

(11) **EP 1 100 507 B1**
(45) Date of publication and mention of the grant of the patent: **24.01.2007**
(21) Application number: 99937735.1
(22) Date of filing: 02.08.1999
(51) Int. Cl.: A61K 31/52, A61K 31/505, A61K 31/355, A61K 31/375, A61K 31/56, A61P 25/28

(54) **USE OF A COMBINATION COMPRISING A PRECURSOR OF URIC ACID AND ANTIOXIDANT FOR THE TREATMENT OF NEURODEGENERATIVE DESEASES**
EIN PRÄKURSOR DER HARNSÄURE UND EIN ANTIOXIDATIONSMITTEL ENTHALTENDE ZUSAMMENSETZUNG UND DEREN VERWENDUNG ZUR BEHANDLUNG VON NEURODEGENERATIVEN ERKRANKUNGEN
COMBINAISON CONTENANT UN PRECURSEUR DE L'ACIDE URIQUE ET UN ANTIOXIDANT ET SON UTILISATION POUR LE TRAITEMENT DE MALADIES NEURODEGENERATIVES

(30) Priority: 31.07.1998 US 127184
(43) Date of publication of application: 23.05.2001
(73) Proprietor: Sacks, Meir S., Pittsburgh, PA 15217 (US)
(72) Inventor: VAN DYKE, Knox, Morgantown, WV 26505 (US); Sacks, Meir S, Pittsburgh, Pennsylvania 15217 (US)
(74) Representative: Howden, Christopher A.
(86) International application number: PCT/US1999/017463
(87) International publication number: WO 2000/006171

(56) References cited:
- EP-A- 0 113 162
- EP-A- 0 416 248
- WO-A-00/00212
- WO-A-98/00101
- FR-A- 2 692 784
- US-A- 4 758 553
- US-A- 5 043 100
- US-A- 5 328 914
- US-A- 5 391 568
- US-A- 5 728 707

## Description

### BACKGROUND OF THE INVENTION

### Field of the Invention

The present invention is directed to an antioxidant composition, more particularly to a mixture of a precursor of uric acid with antioxidants which is effective in treating diseases associated with oxidative damage.

### Background and Description Of The Related Art

Oxidative damage is the cause of many diseases. Proper redox status appears to be a cornerstone of good health. Dietary antioxidants are extensively involved in metabolic process in a human body. Recent aging studies have shown strong association to the outward signs old ages and oxidative damage, suggesting that antioxidants have anti-aging functions. Oxidative damage is found in diseases such as cancer, rheumatoid arthritis, inflammation, artery occlusion, diabetes, neurodegenerative diseases, and age related macular degeneration. Free radicals are known causes of cell oxidative damage. Free radicals, a primary cause of oxidative damage may be generated by environmental radiation, air pollution, inflammation and excessive physical and mental exertion. Free radicals are generated by the species having a free electron and propagate typically by singlet oxygen and hydroxyl free radical or by specific enzymatic reactions. As a free radical acquires an electron from another molecule, the free radical becomes reduced while the other molecule is oxidized and changes its structure and functions. The biological activity of both oxygen-free radical species and related polyunsaturated fatty acid lipid peroxidation products has been well established. For example, the generation of reactive radical species has been found to be involved in the cytotoxic effects of ionizing radiation, various chemotherapeutic agents, and a variety of other biological processes, including aging, and the initiation and promotion stages of experimental carcinogenesis. The generation and release of reactive free radicals in the respiratory burst phenomenon used by various cells of the immune system is also a well known mechanism of foreign target destruction.

Nucleic Acids, proteins, enzymes and lipid molecules are all susceptible to free radical oxidation. Lipid oxidation can cause damage to membrane systems which include cell membranes, membranes of cellular organelles, and membranes in between. Protein oxidation may lead to cell structure damage. Enzyme oxidation may result in changes in metabolic rates. Nucleic acid damage, for example, DNA damage, may lead to cell mutation and cell necrosis. These unwanted cell changes are the etiology of many serious diseases such as cancer, heart attach, cataracts, neurodegenerative diseases, macular degeneration, and inflammatory diseases.

In aerobes, a variety of radical scavenging mechanisms have evolved at both the cellular and organism level that confer protection from potentially lethal reactive oxygen species, such as the hydroxyl radical, superoxide anion, and hydrogen peroxide. Importantly, oxygen radicals can initiate longer-lived chain reactions of lipid peroxidation that can be propagated from cell to cell. As discussed above, these peroxidation products are capable of damaging cellular DNA, RNA, protein, and cellular phospholipids. Free radicals can undergo a variety of different reactive once they are generated: they can react with another non free radical creating a new free radical; they can react with the same free radical creating a new non free radical; they can react with a different free radical to forma new non radical; a free radical can react with unsaturated lipid to form a lipid peroxide; and a free radical can react with another free radical to produce annhilation. The result of these various reactions can lead to more or less toxic molecules being produced. The protective cellular mechanisms against this kind of damage include anti-oxidants and radical scavengers in both the lipid (e.g., α-tocopherol, β-carotene) and aqueous (e.g., glutathione and ascorbic acid) phases of cells, as well as enzymes such as superoxide dismutase and catalase. High plasma uric acid level found in humans has also been shown to be a free radical protective factor. Inhibition of free radicals is postulated as the way in which certain radioprotectors, such as the free radical scavengers cysteine, glutathione, and related cellular sulfhydryl compounds operate. Glutathione becomes oxidized to contain a dithio group as well as to protein-mixed disulfides, when cells are exposed to oxygen-generating compounds or other oxidative stresses.

Vitamin E is the most well-studied antioxidants. Recent studies confirm that vitamin E supplementation reduces the risk of heart disease. Many epidemiological studies reported the negative association between cancer incidents and the consumption of antioxidants from fruits and vegetables.

Quercetin and epigallocatechin gallate are both antioxidants extracted from plants. Quercetin is a common flavonoid antioxidant found in a variety of fruit and vegetables including, onion, leak, asparagus, cabbage, mustard, ornamentals, pepper, endive, grapefruit, lettuce, apple, mango, plum, radish, black currant, potato, spinach, grape and green tea. Epigallocatechin gallate (EGCg) is an antioxidant extracted from green tea. These antioxidant have strong chemopreventive effect.

U.S. Patent 4,758,553 discloses nutritional supplements for administration to patients in situations in which it is difficult or impossible for the patient to take a diet or nutrients orally. The nutritional supplements proposed comprise nucleic acid components, such as inosine-monophosphate, guanosine-monophosphate, cytidine-monophosphate, uridine-monophosphate, thymidine, inosine, cytidine, uridine, hypoxanthine and vitamins such as vitamin A, B, B₂, B₆, nicotinic acid, pantothenic acid, vitamin C, D, E, biotin, folic acids, etc.

EP-A-0113162 discloses a therapeutic composition useful for the treatment of cancer, comprising a dsRNA in combination with vitamin A or vitamin C.

WO 00/00212 discloses; a composition to be used in the treatment of conditions involving the pulmonary system, comprising a NO inhibitor and xanthines.

EP-A-0416248 discloses the use of the xanthine bronchodilator to be used together with vitamin B₆ in the treatment of obstructive pulmonary diseases.

U.S. Patent 5,328,914 discloses the use of compositions containing xanthine bases and antioxidants to treat hair loss by topical application.

WO 98/00101 discloses cosmetic compositions containing xanthines and xanthine derivatives and an antioxidant agent, for skin tone Improvement.

There exists a need for antioxidant compositions or mixtures thereof whether simultaneously, concomitantly, or consecutively administered which protect, prevent, treat, or ameliorate diseases associated with oxidative damage.

Disclosed herein are compositions and mixtures having oxidative effects against free radical damage. The composition is useful in the prevention or treatment of diseases associated with oxidative damage.

### SUMMARY OF THE INVENTION

According to the present Invention, there is provided use of a composition comprising a precursor of uric acid selected from hypoxanthine xanthine, inosine adenosine guanosine, AMP, GMP, IMP, adenine, guanine or combinations thereof, in the manufacture of a medicament for administration with one or more of the following antioxidants:
(a) vitamin C or vitamin E,
(b) an inhibitor of homocysteine formation selected from vitamin B6 or folic acid,
(c) quercetin, catechin, epicatechin, gallocatechin, epigallocatechin, epicatechin gallate, epigallocatechin gallate, or catechin gallate, or
(d) an extract of green tea,
for the treatment of Alzheimer's disease or neurodegenerative disease.

According to this aspect of the present invention, there is also provided use of one or more antioxidants selected from:
(a) vitamin C or vitamin E,
(b) an inhibitor of homocysteine formation selected from vitamin B6 or folic acid.
(c) quercetin, catechin, epicatechin, gallocatechin, epigallocatechin, epicatechin gallate, epigallocatechin gallate, or catechin gallate, or an extract of green tea,
for the manufacture of a medicament for administration with a precursor of uric acid selected from hypoxanthine, xanthine, inosine, adenosine, guanosine, AMP, GMP, IMP, adenine, guanine or combinations thereof, for the treatment of Alzheimer's disease or neurodegenerative disease.

Disclosed herein is a composition comprised of a therapeutically effective concentration of a precursor of uric acid, selected from the group consisting of DNA, RNA. nucleotides of DNA, nucleotides of RNA. nucleosides of DNA, free bases of DNA, free bases of RNA, hypoxanthine, and xanthine. The precursor of uric acid is preferably hypoxanthine, and the concentration of hypoxanthine is preferably in a range of 100 mg/day to 25g/day, more preferably 1g/day to 20 g/day, even more preferably a range of 2 g/day to 10 g/day. The compositions may also include a therapeutically effective concentration of a biologically active agent selected from the group consisting of a primary antioxidant, a secondary antioxidant, an inhibitor of homocysteine formation, an inhibitor of NO synthase, green tea extract and/or a combination of any one or more of the above.

Also disclosed is a composition comprised of both a precursor of uric acid and a biologically active agent. The precursor of uric acid is selected from the group with the precursor uric acid preferably being hypoxanthine. Preferably, the secondary antioxidant is Vitamin C, Vitamin E or a combination of the two. The inhibitor of NO syntahse is preferably prednisone, while the inhibitor of homocysteine formation is preferably Vitamin Be or folic acid.

Also disclosed is the use of a therapeutic composition in the manufacture of a medicament for treating or preventing diseased states associated with oxidative damage comprised of a precursor of uric acid, which may also include an antioxidant, preferably selected from the group consisting of DNA, RNA*,* nucleotides of DNA nucleotides of RNA, nucleosides of DNA, free bases of DNA, free bases of RNA, hypoxanthine, and xanthine, most preferably being hypoxanthine. The antioxidant active agent is preferably selected from the group consisting of a primary antioxidant, a secondary antioxidant, an inhibitor of homocysteine formulation, an inhibitor of NO synthase, and a combination of two or more of said primary antioxidant, secondary antioxidant, inhibitor of homocysteines, and inhibitor of NO synthase as described above. The secondary antioxidant is preferably selected from Vitamin C or Vitamin E and, wherein, the inhibitor of NO synthase is prednisone and, wherein said inhibitor of homocysteine formation is Vitamin B₆, wherein said inhibitor of homocysteine formation is folic acid. Said use directed to treating diseased state is diclosed, comprised of administering a therapeutically concentration of a precursor of uric acid alone or with a known biologically active agent as described above, wherein the disease state is selected from the group consisting of Alzheimer's disease and neurodegenerative disease.

Further disclosed is a composition comprising a precursor of uric acid; along with a biologically active agent, said biologically active agent being different from said precursor of uric acid. The precursor of uric acid is selected from the group consisting of DNA, RNA. nucleotides of DNA, nucleotides of RNA. nucleosides of DNA, free bases of DNA. free bases of RNA, hypoxanthine, and xanthine. The precursor of uric acid is preferably hypoxanthine, and the biologically active agent is preferably selected from the group consisting of a primary antioxydant, a secondary antioxidant, an inhibitor of homocysteine formulation, an inhibitor of NO synthase, and a combination of two or more of said primary antioxidant, secondary antioxidant, inhibitor of homocystenes, and inhibitor of NO synthase. Preferably the secondary antioxidant is Vitamin C or Vitamin B or a combination of the two.

The inhibitor of NO synthase is preferably prednisone, while the inhibitor of homocysteine formation is preferably Vitamin B₆ or folic acid. The biologically active agent may include a secondary antioxidant and the inhibitor of homocysteine formation, and the inhibitor of NO synthase. The precursor of uric acid and said biologically active agent may be administrated simultaneously, and the uric acid and the biologically active agent may be administered consecutively. The administration route may be selected from the group consisting of oral, sublingual, transdermal, interveneously and/or topically.

Combination chemotherapy using two or more anti-cancer drugs to treat malignant tumors in humans is currently in use in research and in the clinic. The anti-cancer drugs may be antimetabolites, alkylating agents, antibiotics, general poisons, etc. Combinations of drugs are administered in an attempt to obtain a synergistic cytotoxic effect on most cancers, e.g., carcinomas, melanomas, lymphomas and sarcomas, and to reduce or eliminate emergence of drug-resistant cells and to reduce side effects to each drug.

The present invention is based on the discovery that the therapeutic index of antioxidant compositions can be enhanced in vitro and in vivo systems by concomitantly or separately treating the host with a precursor of uric acid. More specifically, a use of the claimed composition directed to the therapeutic or prophylactic treatment of damage to mammalian hosts caused by free radical production is disclosed, which comprises administering to the host pharmacologically effective amounts of at least one precursor of uric acid (i.e. hypoxanthine) and at least one antioxidant selected from a biologically active compound, a free radical scavenger, or a metabolic inhibitor. The biologically active agent is preferably selected from the group consisting of a primary antioxidant, a secondary antioxidant, an inhibitor of homocysteine, an inhibitor of NO synthase, and a combination of one or more of the above. Examples of other free radical scavenger or antioxidants include, buthionine sulphoximine, vitamin C, indomethacin, ibuprofen, N-acetyl cysteine, or aspirin. In another aspect, a composition is disclosed suitable for administration to mammalian hosts comprising a mixture, in pharmacologically effective amounts, of a precursor of uric acid and a second biologically active compounds, preferably an antioxidant.

When the radicals produce toxic peroxides e.g., peroxynitrite, these substances are thousands of times more toxic than the progenitors from which they originated. These compounds can react for a much longer time since their half lives can be up to millions of times longer than their free radical parents. It is the reaction of these new toxic substances that need to be opposed by antioxidants. DNA, RNA, proteins, lipids and carbohydrates are the targets of these toxic peroxides. The antioxidants are usually phenols, di-, tri- or polyphenols, sulfhydryl compounds e.g., glutathione or n-acctyl cysteine or uric acid. Uric acid is a unique antioxidant in humans, apea, and some Dalmatian dogs due to the fact that it builds up in the blood since the enzyme to degrade uric acid (uricase) is missing. Uric acid is a mainstay of antioxidation in the blood and in cells as well. People that possess low levels of uric acid are at risk if diseases occur in which large amounts of toxic peroxides are produced. But by increasing the precursors of uric acid and other secondary antioxidants the toxicity of excess peroxides may be opposed. In addition, a synergism of antioxidation may occur because the whole of the antioxidation is greater than the sum of its component parts.

### BRIEF DESCRIPTION OF THE DRAWINGS

The features, aspects and advantages of the present invention will become better understood with regard to the following description, appended claims, and accompanying drawings where:
Figure 1 illustrates the metabolic degradation of free based purines to uric acids.
Figure 2 is a graph illustrating the oxidative reaction of peroxynitrite and luminol and the inhibition thereof with uric acid.

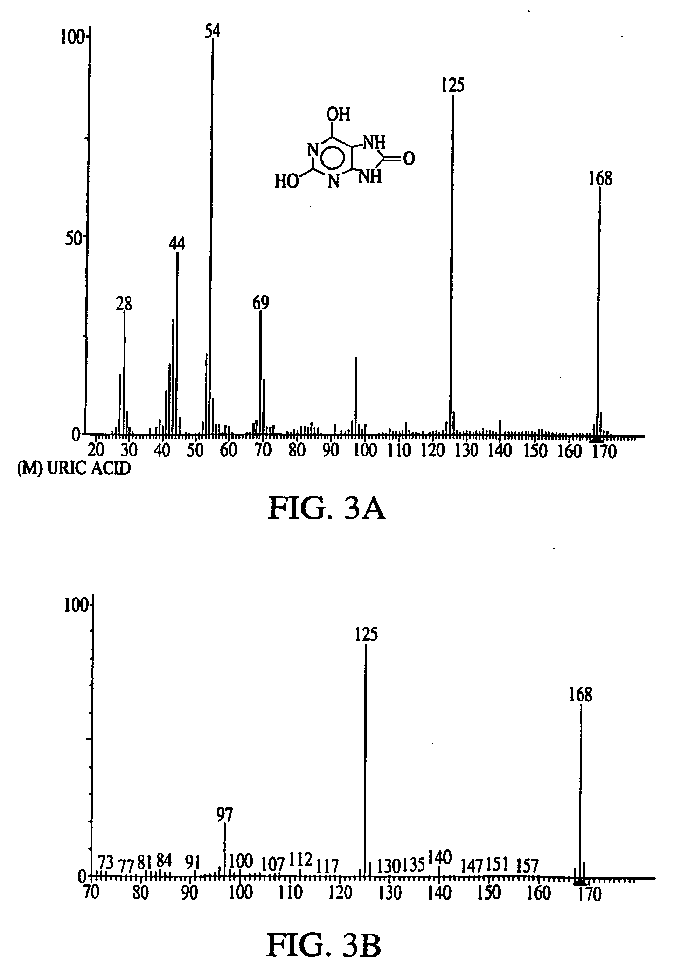

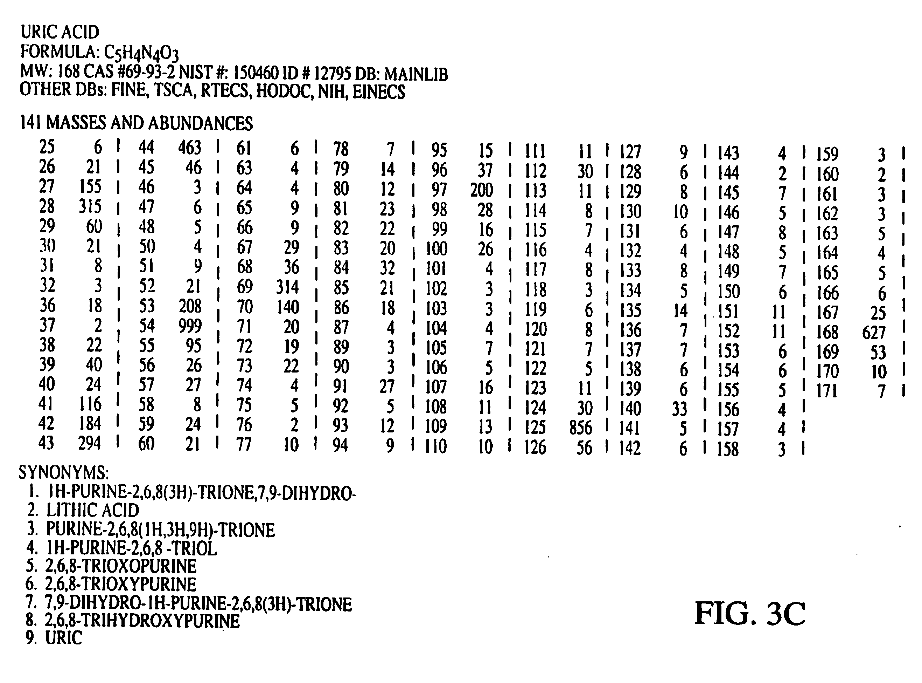

Figure 3 is a mass spectrum of uric acid with various molecular weight fragments identified on the spectrum.
Figure 3A is a fragment chart for uric acid corresponding to the mass spectrum of Figure 3.
Figure 4 is the mass spectrum of allantoin, the oxidated fragment of uric acid when uric acid is treated with peroxynitrite.
Figure 5 illustrates the major polyphenols isolated from green tea extract.
Figure 6 is a graph illustrating the chemiluminescence of various sydnonimine congeners.
Figure 7 illustrates the luminol dependent chemiluminescence of multiple injections of peroxynitrite.
Figure 8 illustrates the inhibitory effect of green tea extract on SIN-1.
Figure 9 illustrates the inhibitory effect of green tea extract on peroxynitrite.
Figure 10 illustrates the inhibitory effect of (-) epicatechin on SIN-1.
Figure 11 illustrates the inhibitory effect of epicatechin on peroxynitrite.
Figure 12 illustrates the inhibitory effect of (±) catechin on SIN-1.
Figure 13 illustrates the inhibitory effect of (±) catechin on peroxynitrite.
Figure 14 illustrates the inhibitory effect of (-) epigallocatechin on SIN-1.
Figure 15 illustrates the inhibitory effect of (-) epigallocatechin on peroxynitrite.
Figure 16 illustrates the inhibitory effect of (-) epicatechin gallate on SIN-1.
Figure 17 illustrates the inhibitory effect of (-) epicatechin gallate on peroxynitrite.

### DETAILED DESCRIPTION OF THE PRESENT INVENTION

As used herein, the term "pharmaceutically acceptable" refers to a carrier medium which does not interfere with the effectiveness of the biological activity of the active ingredients and which is not toxic to the hosts to which it is administered.

As used herein, the term "about" means plus or minus 10% of the number to which reference is being made. For example, about 10 grams means in the range of 9-11 grams.

As used herein, the term "prophylactic or therapeutic" treatment refers to administration to the host of the antioxidant(s) and/or biologically active agent(s) either before or after onset of the biological damage to the host. If the antioxidant(s) or biological agent(s) are administered prior to exposure to the agent causing the biological damage, the treatment is prophylactic (i.e., it protects the host against the damage), whereas if it is administered after exposure to the agent causing the damage, the treatment is therapeutic (i.e., it alleviates the existing damage).

The scheduling and dosing will depend, e.g., on the type of host, disease, and antioxidant(s). If the biological damage is caused by infection, the doses are preferably administered from 18 hours before infection for prophylactic treatment and in early phase of infection for therapeutic treatment, up to 18 hours after infection in later phase of infection for therapeutic treatment. If the biological damage is cancer, the treatment is not considered therapeutic if after treatment a tumor appears or if an existing tumor burden is not eliminated or decreased. The effect of the doses may diminish with time, but for humans the dose may be repeated for months or even years. As used herein, the term "biological damage to the host caused by free radical generation" refers to any cellular, tissue or other damage to body parts or functions sustained by the host as a result of free radicals being produced in the body of the host. Examples by which such damage may be caused include hypothermia, which may occur during cancer treatment as when the temperature of the tumor is increased via local or general microwave irradiation, damage caused by chemotherapeutic agents (chemotherapy), radiation therapy, or high oxygen tension that produce radicals to kill cells, and infection. Also, treated tumor cells may help propagate radical damage.

An example of high oxygen tension is the condition that occurs when premature babies are exposed to high pressure oxygen, resulting in retinal and lung disease. Other conditions that represent damage caused by free radical generation may also be envisioned and fall within this definition.

The term "cancer" as used in the above definition refers to any neoplastic disorder, including such cellular disorders as, for example, renal cell cancer, Kaposi's sarcoma, chronic leukemia, breast cancer, sarcoma, ovarian carcinoma, rectal cancer, throat cancer, melanoma, colon cancer, bladder cancer, mastocytoma, lung cancer and gastrointestinal or stomach cancer. Preferably, the cancer is colon cancer, melanoma, renal cell cancer, sarcoma, lung cancer, adenocarcinoma, or breast cancer.

The term "infection" as used in the above definition refers to any kind of pathogenic disease, including those caused by bacteria, fungi, viruses, protozoa or parasites. Examples of bacterial infections include P. aeruginosa, E. coli, tetanus, Mycobacterium species, Streptococcal strains, diphtheria and Salmonella. Examples of fungal infections include cryptococcosis, histoplasmosis, and other infections due to Candida species. Examples of viral infections include Hepatitis A, recurrent Herpes Simplex, AIDS, Herpes, Zoster, influenza, and rhinoviruses. Preferably, the infection is bacterial, more preferably Gram-negative infection, and most preferably P. aeruginosa and E. coli infection.

As used herein, the term antioxidant unless otherwise distinguished refers to: a free radical scavenger or a metabolic inhibitor. The term "free radical scavenger" refers to any compound or substance that protects a mammalian host against biological damage caused by free radical generation. This definition includes those agents that act through direct radical scavenging as well as those that act by altering the radical scavenging ability of the host and/or tumor. Such free radical scavengers may be radiation protectors, and include such compounds as, for example, hypoxanthine, buthionine sulfoximine, diethyl maleate, vitamin E, vitamin C, cysteine such as N-acetyl cysteine, or glutathione, metronidazole, and a retinoid such as, e.g., vitamin A. Any combination of these modifiers may be employed. Most preferably, the free radical scavenger employed herein for humans is buthionine sulphoximine, vitamin C, vitamin E, or N-acetyl cysteine modifier.

"Metabolic inhibitor," refers to a compound or substance that blocks or inhibits the cyclooxygenase and/or lipoxygenase metabolic pathways, of the arachidonic acid cascade, wherein phospholipids are converted to arachidonic acid, by phospholipase A2 or C, and the arachidonic acid may proceed by either metabolic pathway. Such blockage or inhibition may be of an enzyme that catalyzes one or both pathways, of a cell type that contains the enzyme, or of one or more of the natural products of the pathways. Examples of metabolic inhibitors include aspirin, indomethacin, ibuprofen, nordihydroguaiaretic acid (4,4'-[2,3-dimethyl-1,4-butanediyl]-bis[1,2-benzenediol]) (NDGA), cis-8,11,14-eicosatrien-5-ynoic acid (ETYA), and synthetic (as opposed to natural) prostaglandins and/or leukotrienes that block the effects of the natural metabolic products at the production level rather than at the enzyme level Aspirin, indomethacin, ibuprofen, and ETYA block the cyclooxygenase pathway, thereby inhibiting the production of natural prostaglandins, thomboxanes and prostacyclins. At higher concentrations, indomethacin also blocks phospholipase.

As used herein, the term "pharmacologically effective amounts" as applied to hypoxanthine, antioxidants or biological modifiers refers to the amount of each component in the mixture or administered to the host that results in an increase in the therapeutic index of the host.

The "therapeutic index" can be defined for purposes herein in terms of efficacy (extent of tumor or infection reduction or other cure) and in terms of toxicity to the host. For non-human hosts, if the efficacy increases at least 50% over the efficacy using an excipient control (e.g., phosphate buffered saline) and the ratio of mean body weight at the end of the evaluation period for efficacy response to mean body weight at the start of treatment is at least 0.90 (i.e., no greater than 10% body weight loss), the therapeutic index has increased. The ratio of mean body weights indicates the extent of toxicity, with a value of 1 indicating no toxicity. For non-human hosts being treated for cancer, the extent of efficacy achieved may be measured by the ratio of mean tumor volume at the end of the evaluation period for efficacy response to mean tumor volume at the start of treatment. A reduction in the ratio of at least 50% of treated over excipient control indicates increased efficacy. The most preferred doses, schedules, and types of biological modifiers are those that achieve a mean tumor volume ratio of between 0 and 5, with a value of 0 being optimum and indicating a cure. For human hosts, if the efficacy increases at least 50% upon treatment with the lymphokine/cytotoxin and biological modifiers and the toxicity is acceptable, i.e., no more than fever, chills, and/or general malaise, the therapeutic index has increased. For human hosts being treated for cancer, the extent of efficacy is generally ascertained in the clinic by measuring the perpendicular diameters of the products of all measured disease.

A partial response occurs when the tumor shrinks by at least 50% in the sum of the products of the perpendicular diameters of all measured disease.

The present invention involves administering to a mammalian host, preferably a human host, pharmacologically effective amounts of one or more precursors of uric acid and one or more antioxidants or biologically active agents. The precursors of uric acid (i.e. hypoxathine), antioxidants, and biologically active agents may be combined in vitro before administration or separately administered to the host, in either order or concurrently or simultaneously, with any administration of the precursor of uric acid generally taking place up to 24 hours after the administration of the other antioxidants or biological active agent(s).

The administration(s) may take place by any suitable technique, including oral, subcutaneous and parenteral administration, preferably parenteral or oral. Examples of parenteral administration include intravenous, intraarterial, intramuscular, and intraperitoneal, with intraperitoneal and intravenous being preferred. The dose and dosage regimen will depend mainly on whether the precursor(s), antioxidant(s), and biologically active agent(s) are being administered for therapeutic or prophylactic purposes, separately or as a mixture, the type of biological damage and host, the history of the host, the type of precursored antioxidant or biologically active agents or cytotoxin, and the type of biological modifier employed. The amount must be effective to achieve an enhanced therapeutic index as defined above. It is noted that humans are treated longer than the mice and rats with a length proportional to the length of the disease process and drug effectiveness. The doses may be single doses or multiple doses over a period of several days, but single doses are preferred. For purposes herein, a protection level of at least 50% means that at least 50% of the treated hosts exhibit improvement against the disease or infection, including but not limited to improved survival rate, more rapid recovery, or improvement or elimination of symptoms. The doses may be single doses or multiple doses. If multiple doses are employed, as preferred, the frequency of administration will depend, for example, on the type of host and type of cancer, dosage amounts, etc. For some types of cancers or cancer lines, daily administration may be effective, whereas for others, administration every other day or every third day may be effective, but daily administration ineffective. The practitioner will be able to ascertain upon routine experimentation which route of administration and frequency of administration are most effective in any particular case. The dosage amounts for cancer which appear to be most effective herein are those that result in regression in size of the tumor or complete disappearance or non-reappearance of the tumor, and are not toxic or are acceptably toxic to the host patient. Generally, such conditions as fever, chills and general malaise are considered acceptable. The optimum dose levels will depend on many factors, for example, on the type of host, cancer, route, schedule and sequence of administration, existing tumor burden, the type of precursor and antioxidant modifier, and the definition of toxicity. Toxicity may be defined by the extent and type of side effects in human hosts, with fever, chills and general malaise considered acceptable toxicity for the study herein, or by the amount of body weight loss or by death in non-human hosts after a certain period of time, as defined above for the therapeutic index.

Peroxynitrite (OONO-) has recently become clearly associated with a variety of degenerative diseases including Alzheimer's (1), multiple sclerosis (2), Parkinson's, sepsis, kidney toxicity, meningitis, stroke, and a variety of inflammatory based diseases eg. diabetes and arthritis (3). This strong oxidizer, which causes hydroxylation and or nitration of many organic targets but especially phenols and sulfhydryl containing compounds, is produced by macrophages in a variety of tissue locations including the brain and the skin. It is formed from the free radicals superoxide (0-0)- and nitric oxide (NO) each of which has an unpaired electron. The unmatched electrons create a strong affinity which causes the two to bond and to form peroxynitrite. Peroxynitrite is a unique substance because it is a very powerful oxidant which can cause damage to all major types of biochemical entities e.g. DNA, RNA, proteins, carbohydrates and lipids. It is 1000 times more active as an oxidizer than hydrogen peroxide on a basis of molecular weight comparison. Its half life in physiological fluid is about one second.

Endogeuous nitric oxide can be formed from, for example, the oxidative breakdown of 1-arginine or in the presence of hydrogen peroxide or other oxidants or enzymatically from NO synthase. Three types of NO synthase are known of which type two is inducible e.g. stimulation of macrophages with interferon gamma, virus, bacteria or foreign particles increases the formation of the enzyme. The nitric oxide synthase reacts with 1-arginine and forms citrulline and nitric oxide. This allows the macrophage to play a pivotal role in immune mechanisms by producing large amounts of NO which leads to the creation of peroxynitrite and ultimately to the inactivation of the invader. However, if this mechanism is inappropriately activated, peroxynitrite attacks any of biochemical entities which it contacts - including DNA, RNA, proteins and lipids - thus creating pathological damage. Therefore control of the production of NO is critical.

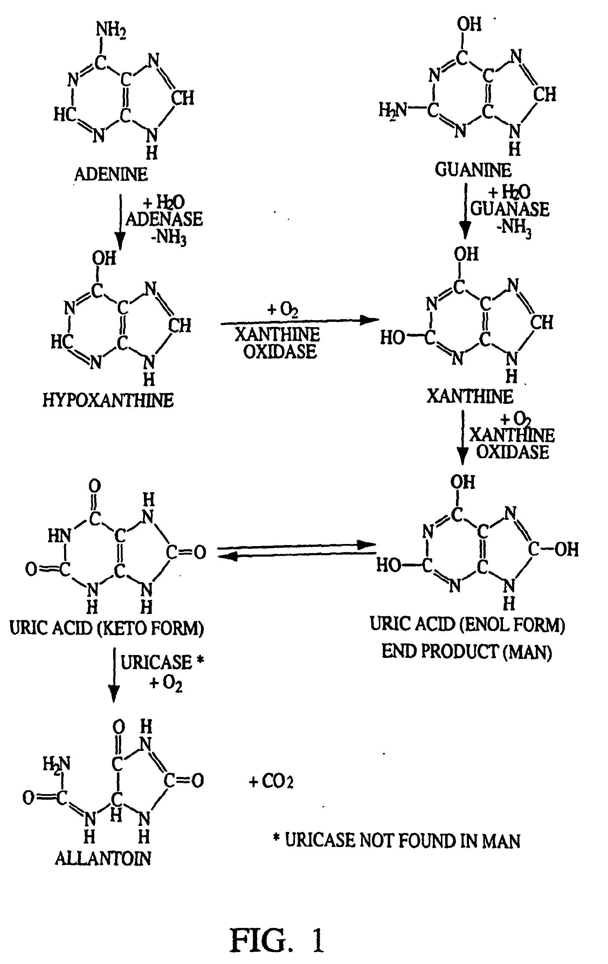

It has been reported that a pharmacological dose of anti-inflammatory steroid can block the induction of nitric oxide synthase. This treatment, however, does not address any peroxynitrite already being formed in great quantities. In this case, peroxynitrite must be scavenged. Therefore an inhibitor or its precursor must be available both inside and outside cells in sufficient quantities and possess a strong reactivity with OONO- in order to inhibit toxicity of peroxynitrite by acting as an antioxidant against the peroxynitrite thus destroying it as an oxidant and metabolizing the antioxidant. Uric acid, seen in Figure 1, is present in blood and tissues in sufficiently high amounts and it represents a chemical structure which is highly vulnerable to attack by strong oxidants like peroxynitrite.

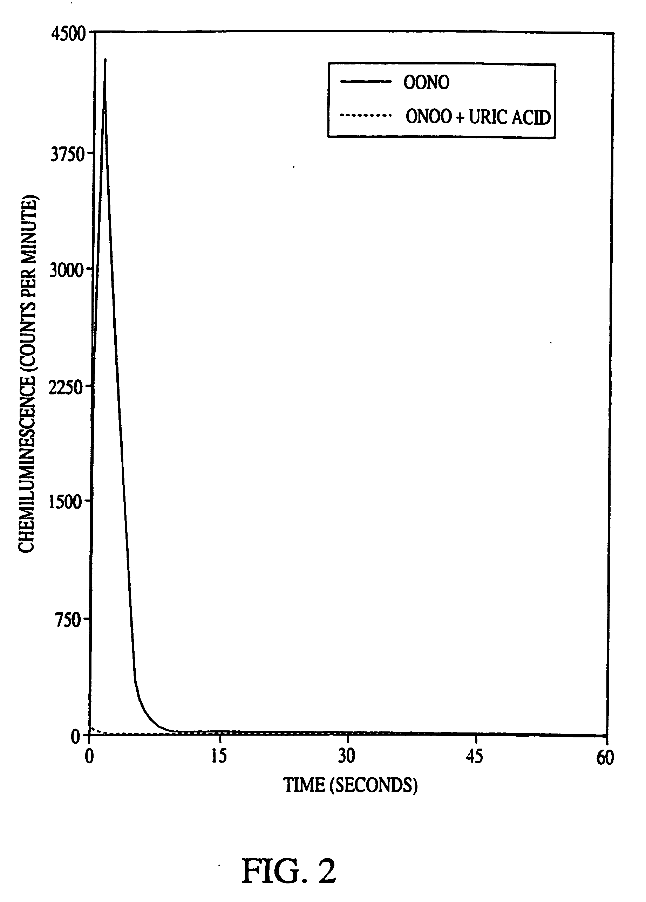

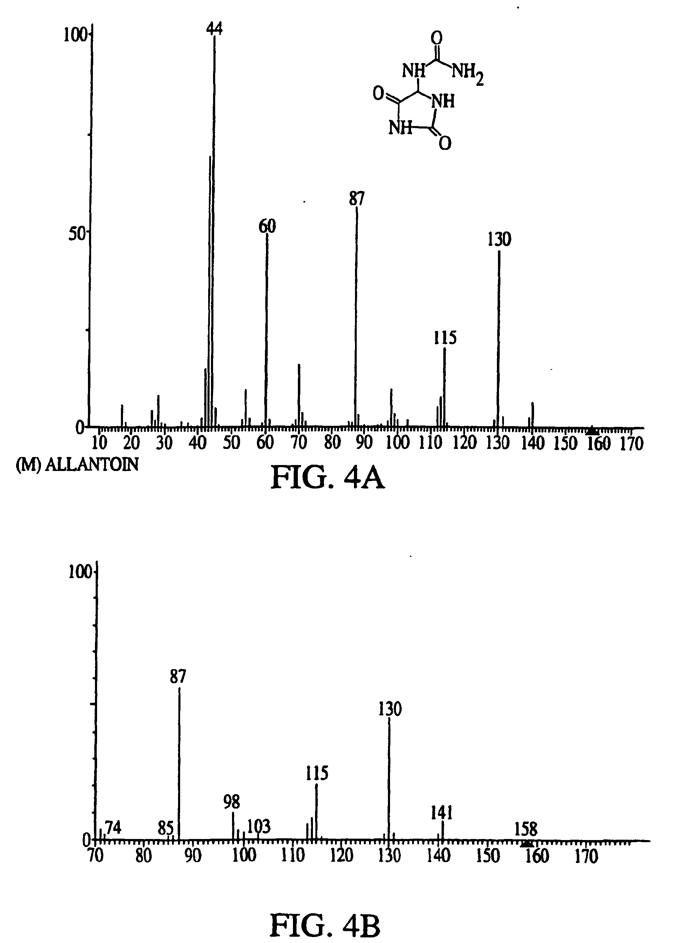

The end product of purine metabolism in humans is uric acid. This substance is an excellent antioxidant that can interact directly with nearby peroxynitrite. Figure 2 is a graph depicting interaction of peroxynitrite and uric acid and the inhibition of peroxynitrite based chemiluminescence. Figure 2 illustrates the oxidative reaction of peroxynitrite an luminol to produce light (chemiluminescence) and the interference with light which is produced by uric acid at a concentration of 10 µM. The proxynitrite concentration is also 10 µM and an inhibition of the oxidative reaction up to about 99% demonstrates the antioxidant potency of the uric acid. During this direct chemical reaction, uric acid is converted to allantoin - a reaction that does not occur in man by enzymic catalysis because the key enzyme uricase is missing. The reaction of uric acid with OONO-, the peroxynitrite, causes uric acid to be chemically changed to allantoin (5-ureidohydantoin) as the mass spectroscopy data in Figure 4 shows. Allantoin is the end product of purine metabolism in animals except for man, great apes and some dalmatian dogs. Both humans, apes and dalmatians lack the enzyme uricase so that the presence of allantoin in these beings indicates that a strong oxidant has attacked the uric acid. Such an oxidant occurs most likely through peroxynitrite or possibly hypochlorite.

Recently a disease very similar to multiple sclerosis, termed allergic encephalomyelitis (EAE), was treated with uric acid. Uric acid is a natural scavenger of peroxynitrite and which is crucial to preventing the damage formed in EAE. Scientists treated the mice with 500 mg/kg of uric acid four times a day and produced long time survival regardless of whether the treatment was initiated before or after the clinical symptoms of EAE had appeared. In addition, evaluation of 20 million patient records for the incidence of multiple sclerosis and high levels of uric acid in the blood reveal that the two diseases are almost mutually exclusive. This raises the clear possibility that hyperuricemia may protect against multiple sclerosis. Uric acid degradation has been implicated in chronic lung disease in infants and in Wilson's disease, an inherited disease of copper metabolism. Uric acid levels are seen to decrease in insulin-dependent diabetes mellitus. Uric acid appears to be such an important as an antioxidant in the body of humans that accounts for almost the total antioxidant capacity of human serum from the blood, according to recent data from Ryan, Grayson, and, Clarke in Ann. Clin. Biochem. 34, 688-689, 1997.

Uric acid also an important role in the tissues although it probably is not as dominant an oxidant in the tissues because of the presence of glutathione. Maintaining the level of uric acid in the body was thought to be mainly under genetic control and couldn't be influenced to any substantial degree by diet, tissue death or turnover. Our studies indicate that while uric acid may be under genetic control, diet can influence the level of uric acid in the blood.

However, uric acid has some obvious disadvantages as a "drug." It is highly insoluble in water and therefore is expected to exhibit poor bioavailability. One of the primary factors weighing against its use as a drug is then elevated blood levels can precipitate an attack of gouty arthritis. Also, high levels can also cause kidney toxicity and possibly kidney failure.

Accordingly, it is suggested herein that the best method to raise the level of uric acid to protect humans against oxidative damage is to ingest close precursors of uric acid that are relatively soluble which would cause the least toxicity. Some precursors, however, pose significant risks. When administered in large amounts (95 mg/kg), adenine can be oxidized to a 2,8 dihydroxyadenine which can crystallize to form kidney stones.

Dietary DNA or RNA is another possibility with doses of 2-4 grams of extract per day. This regimen can boost uric acid levels from normal (4.9 mg in 100 ml of blood) to hyperuricemic (7.5 mg in 100 ml of blood) and thus produce gout. Additionally, RNA and DNA both contain ribose and deoxyribose sugars and phosphates which are not necessary to supply purines resulting in the need to administer a larger quantity of nutritional precursor. The nucleotides AMP, GMP, and IMP were compared to the nucleosides (non-phosphorylated bases with ribose attached) adenosine, guanosine and inosine. They were equivalent in terms of their abilities to increase uric acid levels. The nucleotides AMP and GMP are more effective than DNA and RNA in increasing blood uric acid levels. This suggests the limiting factor for dietary absorption is the rate of hydrolysis (breakdown) of nucleic acids. The uricosuric effect of adenine, guanine, hypoxanthine, xanthine, AMP, GMP and was compared in normal, hyperuricemic and gouty humans. AMP, GMP, hypoxanthine and adenine raised uric acid levels in the blood while guanine and xanthine were ineffective in all three groups.

Of AMP, GMP, and hypoxanthine, overall, hypoxanthine (see Fig 1) appears to be the most effective precursor of uric acid.

Orally administered hypoxanthine appears to be the most effective method to raise the level of uric acid in the blood. Other routes of administration are also possible with the possible exception of inhalational. Any form of injection of these substances would be effective as would be suppository via rectum. Additionally, an intranasal or an exchange dialysis route might be effective.

In the preferred embodiment of the initial level of uric acid in blood and urine would be measured prior to treatment with uric acid precursors. Hypoxanthine or other aforementioned precursor of uric acid would be then administered daily by mouth or other routes and at doses which would probably range from 2-10 grams per day. Subsequently, the level of uric acid in the blood would be assessed to verify it remained to equal or less than 7 mg/100ml. This prevents excessively hyperuric acid levels in order to prevent gout. Due to biological differences in individuals, individual patients should be titrated. In some, daily doses above or below 2-10 grams may be required in order to achieve the therapeutic level of uric acid. In some clinical situations, excessive peroxynitrite levels may consume the additional uric acid necessitating an increased dosage.

In addition to uric acid, a variety of antioxidants are biologically important as therapeutic adjuncts which assist the uric acid in protecting the body against strong oxidants. Increasing the levels of these other antioxidants supports the actions outlined above. These antioxidants include: ascorbic acid (vitamin C and non-toxic derivatives e.g. ester C, Ca++ ascorbate), vitamin E (a-tocopherol or equivalent) and precursors that boost the amounts of glutathione in a non-toxic fashion e.g. n-acetyl-1-cysteine. These three form a cycle in which vitamins C and E protect glutathione so that it remains in a reduced state. The dose of each will vary according to the pathology, but the purpose in each case is to assist the increased uric acid levels in protecting from damage by peroxynitrite and other strong oxidants.

Small doses of a short-acting anti-inflammatory steroid (e.g. prednisone) may also be added to this regimen administered on alternate or every third day. The steroid functions to inhibit inducible NO synthase and thus to ultimately decrease the formation of peroxynitrite. The dose should be kept low to prevent suppression of natural glucocorticoids produced by the adrenal. Other steroidal anti-inflammatory drugs could be substituted (for example those Goodman and Gilman 9th ed. pp. 1473-74-table 59-4).

Doses in the range of 2 to 3 times the normal daily requirement (NDA) of Vitamin B₆ and Folic acid may be added to inhibit the formation of homocysteine. This substance is a possible metabolite of n-acetyl-1-cysteine and is potentially dangerous in long term usage without the addition of B6 and folic acid.

Green tea leaf extract also appears to be ideally suited as an antioxidant or biologically active agent, which alone or combined with a precursor of uric would produce beneficial antioxidative effect. Eppigallocatechin gallate and quercetin are polyphenolic antioxidants found in green tea leaf extract. Quercetin is extracted from rinds and barks of several plants and also from clover blossoms and ragweed pollen. Green tea consumption has been associated with low cancer risks and long life span in epidemiological studies. In vitro studies showed EGCg has chemopreventive properties. Quercetin protects DNA from oxidative damage and inhibits protein kinase activities. These results imply that EGCg and quercetin have potential therapeutical value for the health maintenance and disease prevention.

We studied the effect of Green tea leaf extract on peroxynitrite. This study is based on a simple chemical interaction of peroxynitrite (OONO-) and luminol which produces blue light upon oxidation. Since peroxynitrite has a half life of less than a second, a drug known as SIN-1 is used as a peroxynitrite generator. In addition peroxynitrite itself was used directly with a fast injection-mixing system to ascertain if there might be differences between it and the peroxynitrite generating system (SIN-1) which mimics the natural production of (OONO-). Peroxynitrite is a potent oxidizing compound(1000 times more active than equidose hydrogen peroxide) and it can oxidize carbohydrates, lipids, proteins and nucleic acids. Upon the stimulation of inflammation and/or infection, macrophages and neutrophils can be stimulated to produce large amounts of peroxynitrite.

The health-promoting benefits of tea have been observed and used by the Chinese and Indians for thousands of years. Green tea and black tea are derived from the same plant *(Camellia sinensis).* The difference between the two teas lies mainly in the processing. Green tea is steamed lightly, which inactivates the phenol oxidases responsible for the oxidation of the polyphenols, leaving more of the compounds in an unoxidized state. Therefore, because the polyphenols are left in the reduced or unoxidized state they could act aggressively as antioxidants against strong oxidizing substances like peroxynitrite (P) or even hypochlorite (H) which is produced in the presence of hydrogen peroxide, chloride ion, via the enzyme, myeloperoxidase. When these oxidizing substances are inappropriately spilled outside the phagocytic vacuole they can damage surrounding tissue causing oxidative-based inflammatory diseases eg. Alzheimers, Parkinsons, Amyotrophic lateral sclerosis, stroke, arthritis, anoxia, lung fibrosis, chronic obstructive pulmonary, atherosclerosis, and many other diseases. Therefore, having easily oxidizable substrates such as polyphenols to act as bystander molecules to eliminate the effect of the strong oxidizer, seems appropriate. There is evidence that the polyphenols in tea may act in this manner and protect against antibacterial and/or antiviral activity and oppose cancer and mutation.

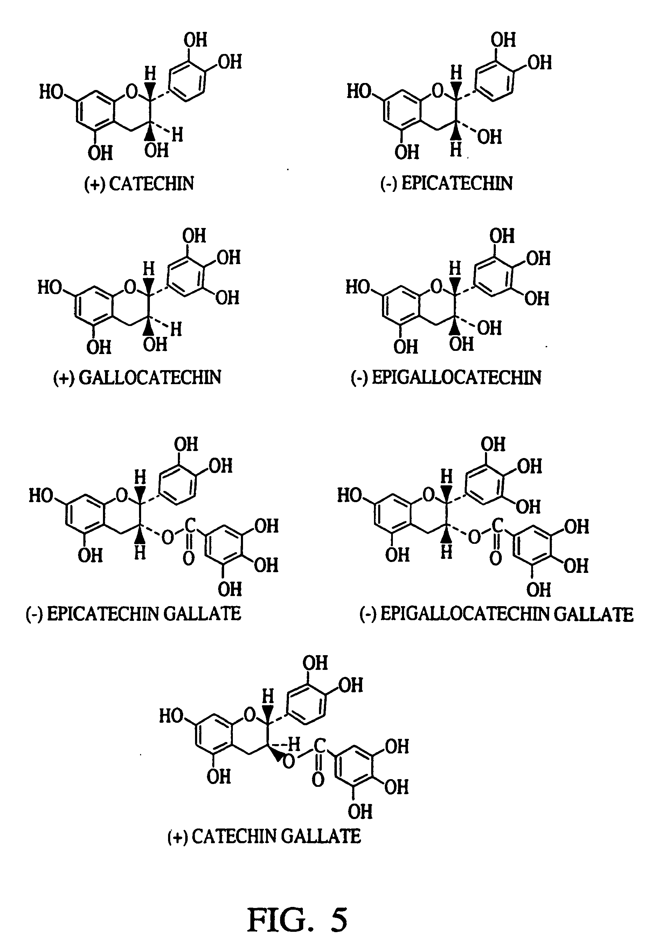

The major polyphenols from green tea are listed in Figure 5. It is thought that the most active antioxidant is Epigallocatechin gallate which is the gallate ester of epigallocatechin. The antioxidant effect of green tea and some of its constituents against luminol dependent chemiluminesence activated by peroxynitrite. Since peroxynitrite oxidizes luminol to an intermediate that produces light, antioxidants will inhibit this blue light by interfering with the oxidation of the luminol. In addition, we used SIN-1 which produces both superoxide and nitric oxide which combine to produce peroxynitrite. The antioxidants in green tea should produce inhibitory activity against the production of light in a similar manner regardless of whether peroxynitrite comes directly or indirectly from SIN-1. However, the components of peroxynitrite ie. superoxide and nitric oxide are produced by SIN-1 and their processing/production or assemblage can be inhibited or interfered in a manner unavailable to peroxynitite alone. The purpose of this study was to measure the antioxidant activity of green tea extract and some of its prominent polyphenols. This is accomplished by using the antioxidants to oppose the oxidation of luminol by peroxynitrite thereby inhibiting the production of blue light.

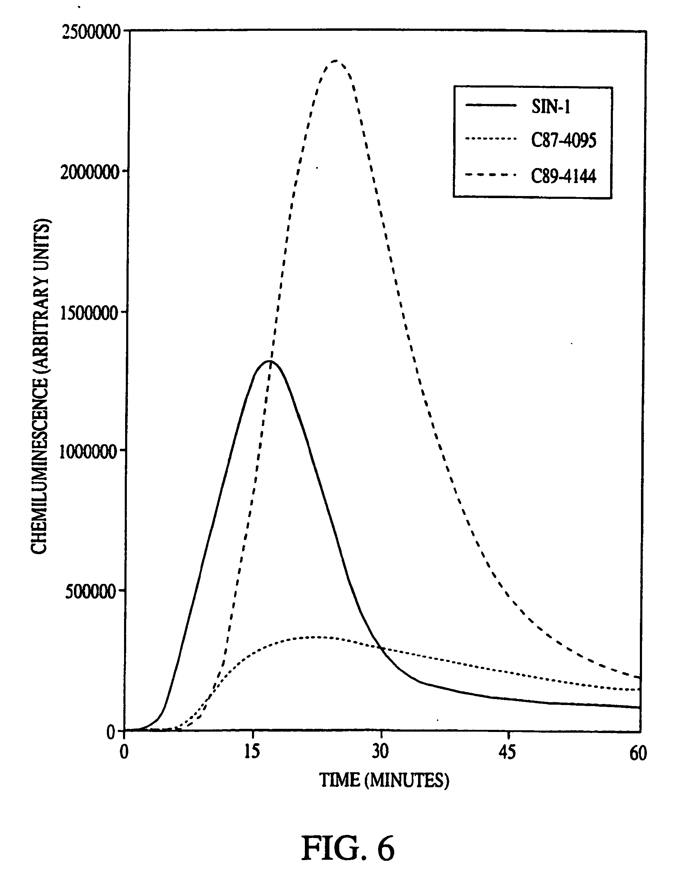

Three sydnonimine congeners are known to produce both nitric oxide and superoxide and then combine to produce peroxynitrite (ONOO). We chose SIN-1 (Linsidomine) over C89-4144 or C87-4095 because it was most efficient in rate of reaction although for total light production C89-4144 was somewhat better (See Figure 6). SIN-1 formed the basis of our continuing experiments and all sydnonimines were obtained from Dr. Karl Schonafinger, Casella-AG Hanauer Landstrasse 526-6000 Frankfurt am Main 61, Frankfurt, Germany. SIN-1 produces peroxynitrite at a rate of about 1% per minute (based on the total amount of compound). SIN-1 was dissolved in 0.1 M PBS buffer (pH 7.4) at a concentration of 15 mg/5ml. It was kept on ice and maintained its activity for several hours of the experiment. Peroxynitrite (approx 50 mM) was diluted 1/1000 with water. The original solution is supplied in 0.3M NaOH and this stabilizes the compound. Once diluted it must be kept on ice and used quickly, especially after dilution. When the diluted OONO- is injected into the PBS buffer at pH=7.4. Rapid injecting and mixing are crucial in this situation because under the neutralizing conditions the half life of OONO- is less than a second, unlike the slower reaction with SIN-1. However, the slower reaction with SIN-1 gives an opportunity to dissect the reaction into its component parts, since rapid reaction and injection/mixing are not so crucial.

The luminol was obtained from Sigma Chemical Company, St. Louis, Mo. It was dissolved first in one ml DMSO and then diluted to produce a stock solution (10-4M) in 0.1 M Phosphate buffered saline at a pH of 7.4.

Green tea powdered extract was purchased from Pharmanex, Simi Valley, California. 250mg was dissolved in 10ml of phosphate buffered saline (PBS) with pH of 7.4 and used as a stock solution. Dilutions were made using PBS and samples from this original stock solution were diluted every ten fold from one to a million (six samples).

100 µl of the luminol solution (0.6mMolar-final concentration), 100 µl of dilutions of green tea in O.1M PBS buffer (pH=7.4) and 200 µl buffer alone and 100 µl of the solution of SIN-1 (5.8 mMolar-final concentration) or derivative was pipetted into a 3 ml round bottom luminometer tube resulting in a total volume of 500 µl. A control solution was also used to provide a reference to the light stimulation produced by the combination of SIN-1 and luminol. The final concentration of this solution consisted of 100 µl of the luminol, 300 µl of the buffer, and 100 µl of the SIN-1. SIN-1 or derivatives were kept on ice prior to pipetting and were the last reagent added because of their instability at room temperature.

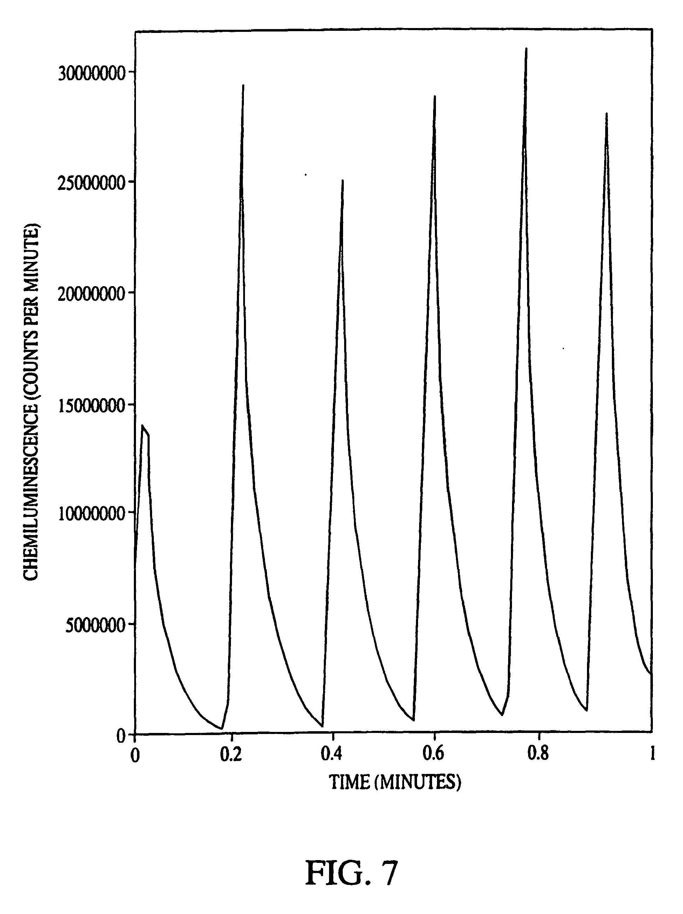

The luminometer tube was placed in a Berthold model LB9505C tube-6 channel luminometer with the temperature control set at 37°C and the light reaction was measured for 20 minutes. Peroxynitrite reacts with luminol directly and was injected using an essentially pressure-independent injection device, which injects 1/50 of it's total volume (2500 µm) of a gas tight syringe, made by the Hamilton Co. (pat. # 3161323) in Whittier, CA, to produce a blue light at 425 nm, which corresponds to the most sensitive detection range of the photomultipliers for this luminometer. The injection device was linked to a gastight 2500 microliter syringe with a fixed needle of 5.2 cm. This length of needle was necessary to penetrate the septum of the ports of the 6 channel Berthold Luminometer and to extend into the tube far enough to produce reproducible mixing necessary because of the short half-life of peroxynitrite. In Fig. 7, repetitive injections of peroxynitrite are shown giving the light generated over a one minute time period which was acquired, plotted and integrated with an IBM clone computer running KINB software. In the case of SIN-1, the ingredients were mixed prior to assay, with SIN-1 added fast. The assay is reported as counts per minute integrated over the 20 minute time period. We measured area under the light curve because it reproduces better and is generally more meaningful than peak height. Area under the curve (AUC) measurements are accomplished by utilizing a KINB program supplied with the instrument (Berthold). This program utilizes trapezoidal approximation as the method of measurement for AUC.

Chemiluminescence was integrated over the initial 20 minutes of each reaction with SIN-1 and one minute for the peroxynitrite. Reactions were repeated in separate experiments a minimum of three times. All bar graphs depict averages and the associated error bars represent standard deviation. Significance level is set at p=0.05 and is calculated from the appropriate student's T test where appropriate. Blanks were run without SIN-1 or peroxynitrite and they produced a trivial relative light signal which was ignored in assays.

Figure 5 shows the structures of the various constituents found in the green tea powdered extract that we assayed. Tests were run to determine the inhibitory effects of the individual constituents on the light producing properties found in the combination of SIN-1/peroxynitrite and luminol. These data form the basis of the results which follow:

The probable mechanism by which SIN-1 hydrolyzes at pH=7.4 and produces the peroxynitrite anion is the result of base catalysis and ring opening of the five membered sydnonimine and its reaction with dissolved oxygen thereby reducing the oxygen and producing superoxide anion and a radical ring opened compound which upon rearrangement releases nitric oxide gas (a free radical). The two free radicals combine and produce the peroxynitrite anion and the spent products. It is the peroxynitrite that reacts with luminol producing blue light which is readily detectable via the photomultipliers of the luminometer.

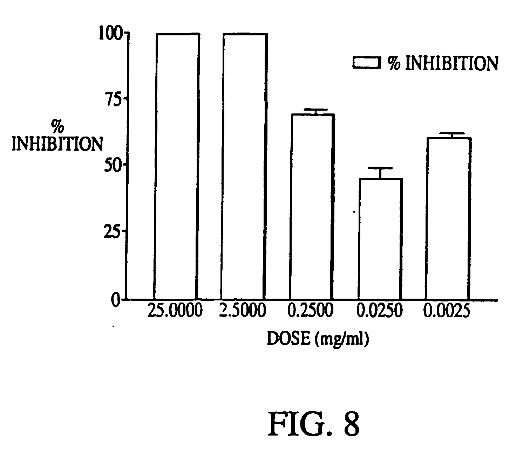

Figure 8 depicts the inhibitory effects of each dilution of green tea when added to the SIN-1/luminol producing combination. The green tea extract consisting of mostly polyphenols produces inhibition of luminol-based light at full strength and up to 10,000 fold dilution and at 0.005mg the inhibition is about 50%. Further dilution appears to produce a slight increase in inhibition.

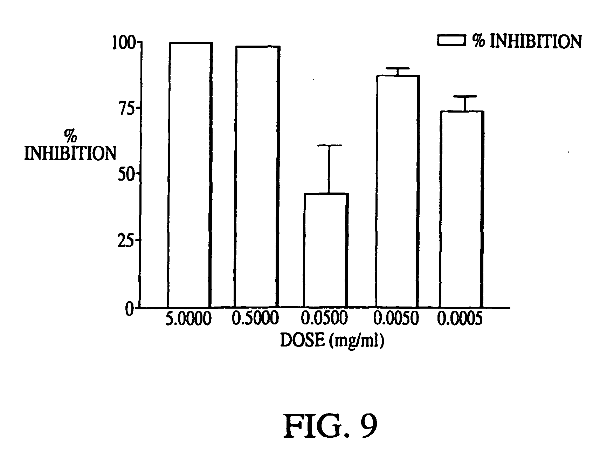

Figure 9 displays a similar inhibition with peroxynitrite and luminol except the 50% inhibition point occurs at a 1000 fold dosage of green tea extract and as the 10 fold dilution continues inhibition increases to 75% or more as sort of a biphasic response.

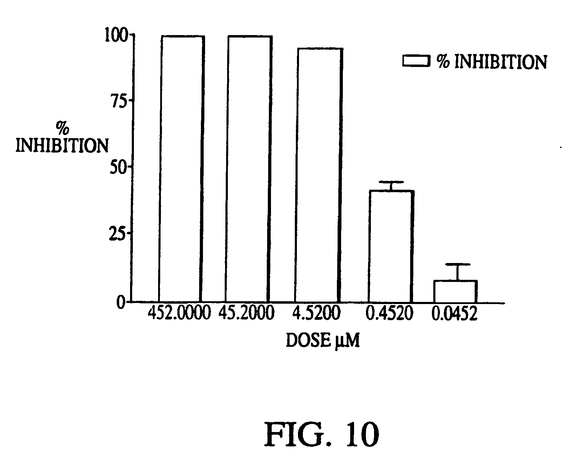

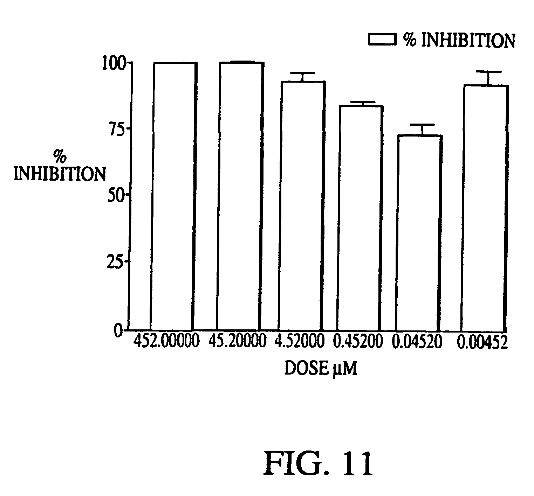

In figure 10 we see the inhibitory effects of (-) epicatechin on SIN-1 activated luminol luminescence. A 1:10,000 dilution at 0.45 µM produces an approximate 10% inhibition. However, in Fig. 11 the same inhibitory potency can be seen with peroxynitrite but at a 10,000 to 100,000 dilution 75-95% inhibition of light can still be seen.

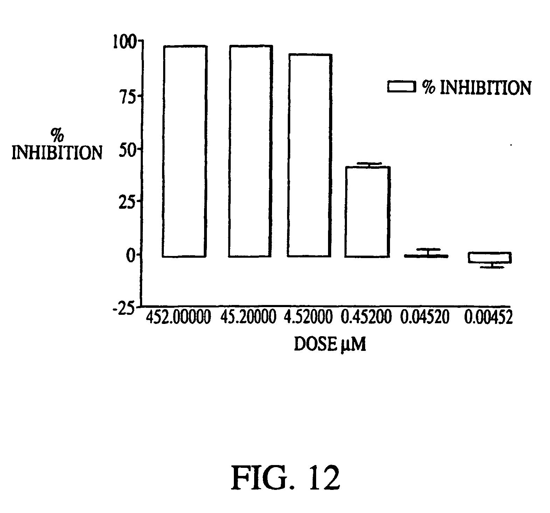

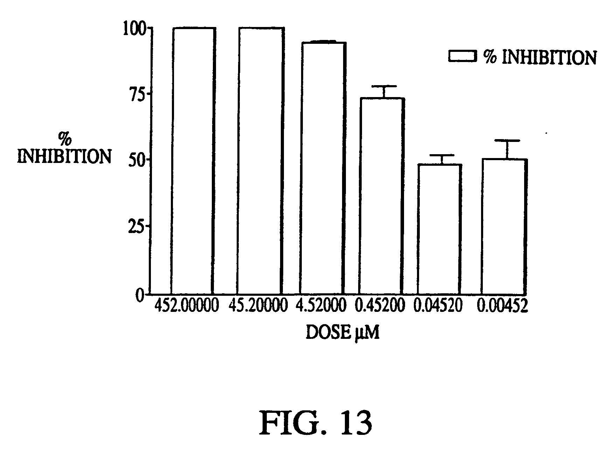

The inhibitory effects of racemic catechin can be observed on SIN-1 activated luminol light. In Fig. 12 at 1:1000 dilution of catechin we observe a 50% inhibition of light but 10 fold dilutions after that display little inhibition. A similar pattern is seen with peroxynitrite-based light in Fig. 13, but the 50% inhibitory effect is not seen until the 1:10,000 dilution. At 1:100,000 dilution, 50% inhibition is still seen.

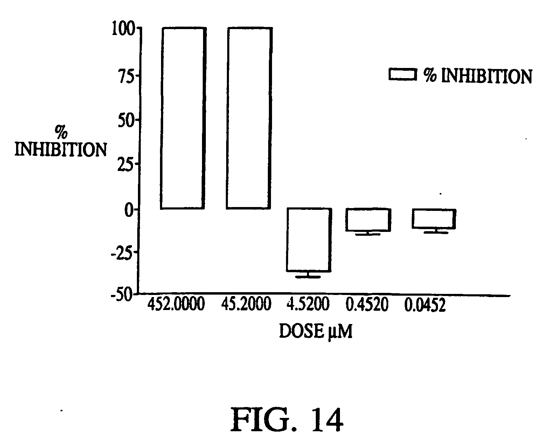

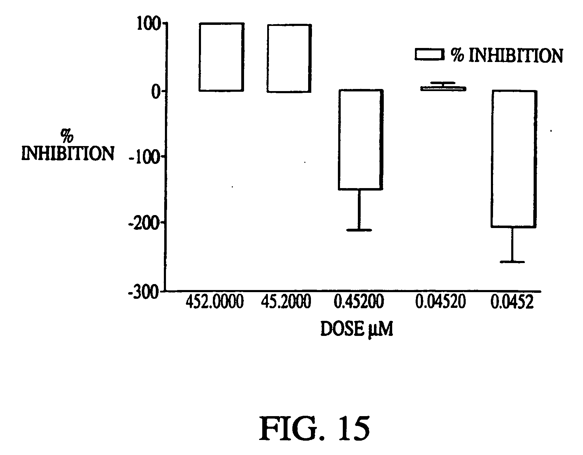

The inhibitory effects on (-) epigallocatechin gallate is seen on SIN-1 activated luminol light in Fig. 14 and it inhibits at full strength and at 1:10 dilution at 100%. but further dilution actually stimulate production of light. The same pattern of behavior can be seen with this compound using peroxynitrite based light in Fig. 15.

As can be seen in Figure 14 epicatechin gallate (-) displayed solid inhibition (60%o) at 1:1,000 dilution when SIN-1 was used as the activator of luminol light but when peroxynitrite was used as the activator of luminol light although 100% inhibition of light was seen at 1:10 dilution about 25% inhibition of light production was seen in consecutive 10 fold dilution after that point (Figure 15).

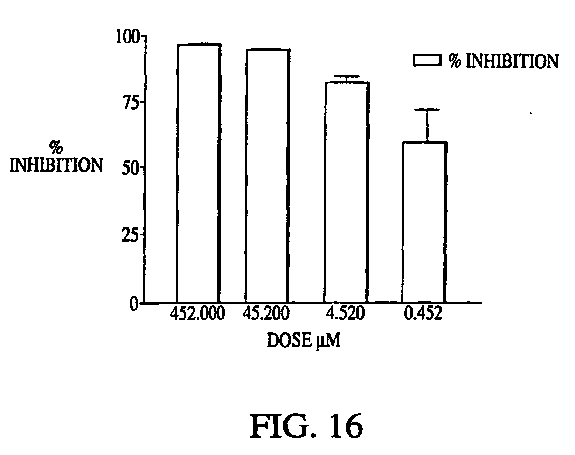

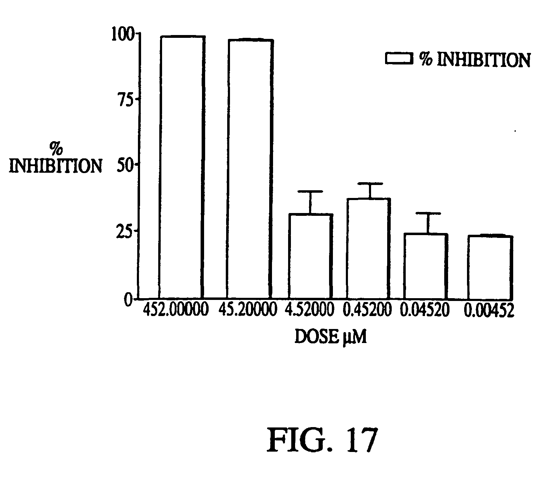

The inhibitory effects on C-1 epicathechin gallate on SIN-1 activated luminol light is shown in Figure 16. A similar pattern is seen with peroxynitrite-based light in Figure 17.

Antioxidants added to the aforementioned reaction mixture will generally interfere with oxidation of luminol by directly reacting with the peroxynitrite. Also, we chose SIN-1 for a source of peroxynitrite because it produced ample light and exhibited the earliest peak. In addition since the components of SIN-1 are produced by this drug, it can be used to generate peroxynitrite at a much slower rate. This allows us the chance to inhibit the individual components of peroxynitrite, similar to how the body might inhibit the actual physiological generation of peroxynitrite.

Extract of green tea inhibits luminol-light production by reacting with peroxynitrite from SIN-1 as seen in (Fig. 8). It inhibits luminol light production from peroxynitrite as well (Fig. 9). Even µM amounts of the tea produce a measurable inhibition. Comparatively, the two sets of figures (Fig. 8 and Fig. 9) should be relatively indistinguishable. The reasons for these discrepancies can only be theorized. One possible explanation to consider concerns the nature of the SIN-1 as compared to the pure peroxynitrite form. SIN-1 degrades over time into peroxynitrite by the formation of superoxide and nitric oxide. Fig. 9 indicates that SIN-1 became less effective the greater the dilutions of tea extract, as expected. However, Fig. 8 indicates that peroxynitrite became less effective at the third dilution, and the further dilutions were demonstrating inhibitory behavior again. It is difficult to explain this phenomena. One possible explanation takes into account the stimulatory capabilities of some phenols. As the structures in Fig. 5 indicate, green tea constituents contain a number of polyphenols. It could be theorized that there is stimulation, as well as inhibition occurring in the tubes containing peroxynitrite rather than SIN-1.

It appears that oxidants play a major role in ridding the body of substances that are unwanted. This includes bacteria, parasites, and viruses. Oxidants such as peroxynitrite, hypochlorite/myeloperoxidase, and superoxide/hydrogen peroxide/ferrous ion can produce high toxicity. When properly confined and compartmentalized, these oxidants play their role in attacking invaders and maintaining health. Sometimes these reactions can get out of control and produce great inflammation which can become chronic e.g. arthritis. Therefore, the chemical reactions involved with colitis, meningitis, multiple organ failure are continually produced in high levels during the actual disease state.

In all these examples of chronic inflammation, toxic oxidants are produced. The oxidants play a major role in producing the signs of inflammation. Therefore, it is logical that chemicals that could keep the oxidants from getting out of control would be antioxidants. There are two well known types of antioxidants-water soluble, e.g. vitamin C, and fat soluble, vitamin E. In addition, the polyphenolic constituents of green tea could be important to spare the antioxidants that are already in the body. Whether a substance acts as an antioxidant or an oxidant depends where the substance in question lies on an oxidant-reductant scale. Ascorbic acid could be an antioxidant or an oxidant depending on where it lies on the scale relative to other substances.

In Figures 10 and 11 we studied the effect of (-) epicatechin on SIN-1 and peroxynitrite activated light respectively. In comparison to the other catechins, the (-) epicatechin constituent from green tea extract demonstrated the overall greatest amount of inhibition of peroxynitrite or SIN-1 based light. Although not wishing to be bound by theory, in reviewing the structures in Figure 5, an explanation could be considered. It contains two diphenolic rings and an alpha OH at the key chiral carbon in the 6 membered cyclic ether. This could allow the easiest access by the attacking peroxynitrite. The second most active antioxidant was (-) epicatechin gallate which is a gallic ester of epicatechin. Its reaction [(-) epicatechin gallate] against SIN-1 or peroxynitrite are seen in Figs. 15 and 16, respectively. In both cases the compound is active in over 1:1000 fold dilution at about 50% inhibition. One would expect similar access to it for peroxynitrite attack as the parent compound above. In addition since it is a gallate ester it might be readily hydrolyzed in the body via esterases, thus releasing (-) epicatechin a gallic acid.

Racemic catechin is very active against SIN-1 or peroxynitrite, as seen in Figs 10 and 11. Indeed, a thousand fold dilution from original stock at).45 mMolar produces about 50% inhibition from both oxidants used previously. This inhibition for peroxynitrite held to 1:10,000 dilution.

Finally the epigallocatechin gallate showed stimulation of light, rather than inhibition, from SIN-1 or peroxynitrite (Figs 13 and 14) at the 1:100 dilution from stock at 0.45 mMolar. Clearly it was the weakest antioxidant tested. One of its phenols has three hydroxyls and of course gallic acid has three phenolic hydroxyls. This combination might allow hindered access toward oxidants. Certainly higher doses of the drug might still be effective. Stimulation of light by phenols or metabolites is well studied phenomenon and recently a chemical logic has been shown in a recent study.

It should be noted that at high dose (-) epicatechin and (-) epigallocatechin gallate changed color when SIN-1 was added to the solution. At the end of the twenty minute incubation in the luminometer the solutions of the two compounds turned a distinct yellow. This color change may have occurred due to oxidative metabolism of the constituents. A possible explanation for this phenomena may be that the constituents experienced nitration and/or hydroxylation.

From the information discussed above, it would appear that a formulation containing a precursor of uric acid/ℓ and/or a compound isolated from green tea extract would be very useful in treating or preventing oxidative damage.

The following table summarizes the proposed treatment regimen. In all cases, the doses are anticipated. The actual dose required will vary with an individual's physiology and pathology.

**Table 1**

| **Substance** | **Dose** | **Purpose** |
|---|---|---|
| Hypoxanthine† | 2-10 g/day | Primary antioxidant |
| Vitamin C | 1-5 g/day | Secondary antioxidant |
| Vitamin E | 1000-3000 IU/day | Secondary antioxidant (fat soluable) |
| n-acetyl-1-cysteine | 500-2000 mg/day | Secondary antioxidant |
| Prednisone | 2-3 times/week | Inhibits inducible NO synthase |
| Vitamin B₆ | 2-3 X NDA*/day | Inhibits homocysteine formation |
| Folic acid | 2-3 X NDA*/day | Inhibits homocysteine formation |

| | | |
|---|---|---|
| † or other precursors of uric acid including DNA, RNA, their nucleotides, their nucleosides, or their appropriate free bases (DNA, RNA) from any biological source. * normal daily requirement | | |

Any or all of the substances that contain purines if ingested in the correct amounts could be used to increase the concentration of uric acid although hypoxanthine is probably the most efficient precursor with the least side effects. This could be used as a time-released substance producing an almost continuous supply of immediate precursor of uric acid. These preferred close ranges can be easily combined with green tea extract.

## Claims

1. Use of a composition comprising a precursor of uric acid selected from hypoxanthine, xanthine, inosine, adenosine, guanosine. AMP, GMP, IMP, adenine, guanine or combinations thereof, in the manufacture of a medicament for administration with one or more of the following antioxidants:
(a) vitamin C or vitamin E.
(b) an inhibitor of homocysteine formation selected from vitamin B6 or folic acid,
(c) quercetin, catechin, epicatechin, gallocatechin, epigallocatechin, epicatechin gallate, epigallocatechin gallate, or catechin gallate, or
(d) an extract of green tea,
for the treatment of Alzheimer's disease or neurodegenerative disease.

2. Use of one or more antioxidants selected from:
(a) vitamin C or vitamin E.
(b) an inhibitor of homocysteine formation selected from vitamin B6 or folic acid,
(c) quercetin, catechin, epicatechin, gallocatechin, epigallocatechin, epicatechin gallate, epigallocatechin gallate, or catechin gallate, or
(d) an extract of green tea,
in the manufacture of a medicament for administration with a precursor of uric acid selected from hypoxanthine, xanthine, inosine, adenosine, guanosine, AMP, GMP, IMP, adenine, guanine or combinations thereof,
for the treatment of Alzheimer's disease or neurodegenerative disease.

3. A use according to Claim 1 or 2, wherein the medicament is also for administration with an Inhibitor of NO synthase selected from an anti-inflammatory steroid or prednisone.

4. A use according to any one of Claims 1 to 3, wherein said precursor of uric acid is hypoxanthine and has a dosage amount of from 100 mg/day to 25 g/day.

5. A use according to any one of Claims 1 to 3, wherein said precursor of uric acid is hypoxanthine and has a dosage amount of from 1 g/day to 20 g/day.

6. A use according to any one of Claims 1 to 3. wherein said precursor of uric acid is hypoxanthine and has a dosage amount of from 2 g/day to 10 g/day.

7. A use according to any one of Claims 1 to 3, wherein said precursor of uric acid is hypoxanthine and has a dosage amount of from 100 mg/day to 10 g/day.

8. A use according to any one of Claims 1 to 7, wherein the precursor of uric acid and the one or more antioxidants are for consecutive administration in either order.

9. A use according to any one of Claims 1 to 7. wherein the precursor of uric acid and the one or more antioxidants are for simultaneous administration.

10. A use according to any one of Claims 1 to 7. wherein the precursor of uric acid and the one or more antioxidants are mixed prior to administration.

11. A use according to any one of the preceding claims, wherein the precursor of uric acid and the one or more antioxidants are for oral, sublingual, transdermal, intervenous or topical administration.

## Patentansprüche

1. Verwendung einer Zusammensetzung, die einen Präkursor von Harnsäure umfasst, ausgewählt aus Hypoxanthin, Xanthin, Inosin, Adenosin, Guanosin, AMP, GMP, IMP, Adenin, Guanin oder Kombinationen derselben, bei der Herstellung eines Arzneimittels zur Verabreichung mit einem oder mehreren der folgenden Antioxidationsmittel:
(a) Vitamin C oder Vitamin E,
(b) einem Inhibitor der Homocystein-Bildung, ausgewählt aus Vitamin B6 oder Folsäure,
(c) Quercetin, Catechin, Epicatechin, Gallocatechin, Epigallocatechin, Epicatechingallat, Epigallocatechingallat öder Catechingallat oder
(d) einem Extrakt von grünem Tee,
zur Behandlung von Alzheimer-Krankheit oder neurodegenerativer Erkrankung.

2. Verwendung eines oder mehrerer Antioxidationsmittel, ausgewählt aus:
(a) Vitamin C oder Vitamin E,
(b) einem Inhibitor der Homocystein-Bildung, ausgewählt aus Vitamin B6 oder Folsäure,
(c) Quercetin, Catechin, Epicatechin, Gallocatechin, Epigallocatechin, Epicatechingallat, Epigallocatechingallat oder Catechingallat oder
(d) einem Extrakt von grünem Tee,
bei der Herstellung eines Arzneimittels zur Verabreichung mit einem Präkursor von Harnsäure, ausgewählt aus Hypoxanthin, Xanthin, Inosin, Adenosin, Guanosin, AMP, GMP, IMP, Adenin, Guanin oder Kombinationen derselben, zur Behandlung von Alzheimer-Krankheit oder neurodegenerativer Erkrankung.

3. Verwendung nach Anspruch 1 oder 2, **dadurch gekennzeichnet, daß** das Arzneimittel auch zur Verabreichung mit einem Inhibitor der NO-Synthase ist, ausgewählt aus einem entzündungshemmenden Steroid oder Prednison.

4. Verwendung nach einem der Ansprüche 1 bis 3, **dadurch gekennzeichnet, daß** besagter Präkursor von Harnsäure Hypoxanthin ist und eine Dosierungsmenge von 100 mg/Tag bis 25 g/Tag hat.

5. Verwendung nach einem der Ansprüche 1 bis 3, **dadurch gekennzeichnet, daß** besagter Präkursor von Harnsäure Hypoxanthin ist und eine Dosierungsmenge von 1 g/Tag bis 20 g/Tag hat.

6. Verwendung nach einem der Ansprüche 1 bis 3, **dadurch gekennzeichnet, daß** besagter Präkursor von Harnsäure Hypoxanthin ist und eine Dosierungsmenge von 2 g/Tag bis 10 g/Tag hat.

7. Verwendung nach einem der Ansprüche 1 bis 3, **dadurch gekennzeichnet, daß** besagter Präkursor von Harnsäure Hypoxanthin ist und eine Dosierungsmenge von 100 mg/Tag bis 10 g/Tag hat.

8. Verwendung nach einem der Ansprüche 1 bis 7, **dadurch gekennzeichnet, daß** der Präkursor von Harnsäure und das eine oder die mehreren Antioxidationsmittel für aufeinander folgende Verabreichung in einer beliebigen Reihenfolge sind.

9. Verwendung nach einem der Ansprüche 1 bis 7, **dadurch gekennzeichnet, daß** der Präkursor von Harnsäure und das eine oder die mehreren Antoxidationsmittel für gleichzeitige Verabreichung sind.

10. Verwendung nach einem der Ansprüche 1 bis 7, **dadurch gekennzeichnet, daß** der Präkursor von Harnsäure und das eine oder die mehreren Antioxidationsmittel vor der Verabreichung vermischt werden.

11. Verwendung nach einem der vorangehenden Ansprüche, **dadurch gekennzeichnet, daß** der Präkursor von Harnsäure und das eine oder die mehreren Antioxidationsmittel für orale, sublinguale, transdermale, intravenöse oder topische Verabreichung sind.

## Revendications

1. Utilisation d'une composition comprenant un précurseur d'acide urique choisi parmi l'hypoxanthine, la xanthine, l'inosine, l'adénosine, la guanosine, AMP, GMP, IMP, l'adénine, la guanine ou des combinaisons de ceux-ci, dans la fabrication d'un médicament pour l'administration avec l'un ou plusieurs des antioxydants suivants :
(a) la vitamine C ou la vitamine E,
(b) un inhibiteur de formation d'homocystéine choisi parmi la vitamine B6 ou l'acide folique,
(c) la quercétine, la catéchine, l'épicatéchine, la gallocatéchine, l'épigallocatéchine, le gallate d'épicatéchine, le gallate d'épigallocatéchine, ou le gallate de catéchine, ou
(d) un extrait de thé vert,
pour le traitement de la maladie d'Alzheimer ou d'une maladie neurodégénérative.

2. Utilisation d'un ou de plusieurs antioxydants choisis parmi :
(a) la vitamine C ou la vitamine E,
(b) un inhibiteur de formation d'homocystéine choisi parmi la vitamine B6 ou l'acide folique,
(c) la quercétine, la catéchine, l'épicatéchine, la gallocatéchine, l'épigallocatéchine, le gallate d'épicatéchine, le gallate d'épigallocatéchine, ou le gallate de catéchine, ou
(d) un extrait de thé vert,
dans la fabrication d'un médicament pour l'administration d'un précurseur d'acide urique choisi parmi l'hypoxantine, la xanthine, l'inosine, l'adénosine, la guanosine, AMP, GMP, IMP, l'adénine, la guanine ou des combinaisons de ceux-ci,
pour le traitement de la maladie d'Alzheimer ou d'une maladie neurodégénérative.

3. Utilisation selon la revendication 1 ou 2, dans laquelle le médicament est destiné également à une administration avec un inhibiteur de synthèse de NO choisi parmi la prednisone ou un stéroïde anti-inflammatoire.

4. Utilisation selon une quelconque des revendications 1 à 3, dans laquelle ledit précurseur d'acide urique est l'hypoxanthine et a une posologie de 100 mg/jour à 25 g/jour.

5. Utilisation selon une quelconque des revendications 1 à 3, dans laquelle ledit précurseur d'acide urique est l'hypoxanthine et a une posologie de 1 g/jour à 20 g/jour.

6. Utilisation selon une quelconque des revendications 1 à 3, dans laquelle ledit précurseur d'acide urique est l'hypoxanthine et a une posologie de 2 g/jour à 10 g/jour.

7. Utilisation selon une quelconque des revendications 1 à 3, dans laquelle ledit précurseur d'acide urique est l'hypoxanthine et a une posologie de 100 mg/jour à 10 g/jour.

8. Utilisation selon une quelconque des revendications 1 à 7, dans laquelle le précurseur d'acide urique et l'un ou plusieurs antioxydants sont administrés consécutivement dans chaque ordre.

9. Utilisation selon une quelconque des revendications 1 à 7, dans laquelle le précurseur d'acide urique et l'un ou plusieurs antioxydants sont administrés simultanément.

10. Utilisation selon une quelconque des revendications 1 à 7, dans laquelle le précurseur d'acide urique et l'un ou plusieurs antioxydants sont mélangés avant l'administration.

11. Utilisation selon une quelconque des revendications précédentes,
dans laquelle le précurseur d'acide urique et l'un ou plusieurs antioxydants sont administrés par voie orale, sublinguale, transdermique, intraveineuse ou topique.
